# EUROPEAN PATENT SPECIFICATION

(11) **EP 0 863 874 B1**
(45) Date of publication and mention of the grant of the patent: **27.12.2000**
(21) Application number: 96938058.3
(22) Date of filing: 31.10.1996
(51) Int. Cl.: C07D 209/52

(54) **PROCESS FOR PREPARING DIOXOAZABICYCLOHEXANES**
VERFAHREN ZUR HERSTELLUNG VON DIOXOAZABICLOHEXANDERIVATEN
PROCEDE POUR PREPARER DE DIOXOAZABICYCLOHEXANES

(30) Priority: 30.11.1995 GB 9524466
(43) Date of publication of application: 16.09.1998
(73) Proprietor: Pfizer Limited, Sandwich Kent CT13 9NJ (GB); Pfizer Research and Development Company, N.V./S.A., Dublin 1 (IE)
(72) Inventor: RAY, Stephen James, Sandwich, Kent CT13 9NJ (GB); RUMPUS, John Arthur, Sandwich, Kent CT13 9NJ (GB)
(74) Representative: Wood, David John
(86) International application number: EP9604782
(87) International publication number: WO9719921

(56) References cited:
- WO-A-93/18001
- WO-A-95/19361

## Description

This invention relates to a process for preparing an exo-compound of the formula (I):- 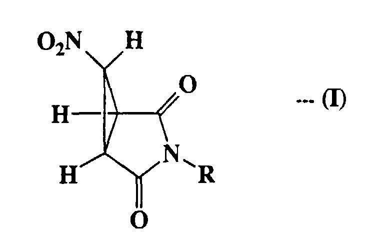 wherein R is C₁-C₆ alkyl, C₃-C₆ cycloalkyl or benzyl, and wherein the phenyl moiety of said benzyl group is optionally substituted by one or more substituents each independently selected from halo, nitro, C₁-C₆ alkyl, C₁-C₆ alkoxy, amino and trifluoromethyl.

Halo means fluoro, chloro, bromo or iodo.

The compounds (I) are useful as synthetic intermediates in the manufacture of the antibiotics of EP-B-0413455 as explained in WO-A-93/18001.

International patent application publication no. WO-A-93/18001 describes a process for preparing a compound of the formula (I) by reaction of a compound of the formula (II):- 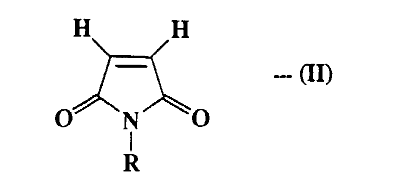 with a halonitromethane in the presence of a base, R being as defined for formula (I).

Example 1 of that application describes the preparation of 1α, 5α, 6α-3-benzyl-6-nitro-2,4-dioxo-3-azabicyclo [3.1.0]hexane by adding the base DBU (1,8-diazabicyclo [5.4.0] undec-7-ene) in toluene dropwise to a mixture of N-benzylmaleimide and bromonitromethane in toluene. However the yield of the end product isolated was only 17%. In terms of grams of activity, the yield would have been less than 17%.

WO-A-95/19361 describes the use of 1,2-dimethyl-1,4,5,6-tetrahydropyrimidine (DMTHP) as the base in that process and the isolated yield was 26.7% (less in terms of grams of activity).

We have now found that if certain specific bases and solvents are used, if the order of addition of the reactants is reversed [i.e., if the halonitromethane and maleimide (II)are added to the base in the solvent], and if any excess base is eliminated from the reaction mixture before recovery of the product (I), then very significant improvements in the yield of (I) are obtained.

Thus the present invention provides a process for preparing a compound of the formula (I):- 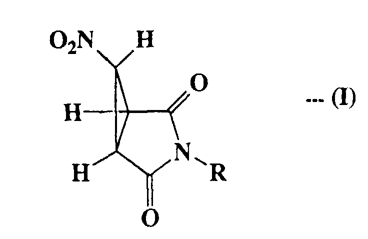 wherein R is C₁-C₆ alkyl, C₃-C₆ cycloalkyl or benzyl, and wherein the phenyl moiety of said benzyl group is optionally substituted by one or more substituents each independently selected from halo, nitro, C₁-C₆ alkyl, C₁-C₆ alkoxy, amino and trifluoromethyl,
which comprises adding a solution comprising a compound of the formula (II), a halonitromethane and an organic solvent selected from acetone, dimethylformamide, dimethylacetamide, acetonitrile, dimethylsulfoxide, N-methylpyrrolidinone and dimethoxyethane, 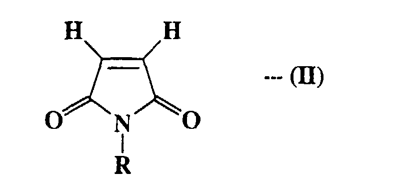 wherein R is as defined above,
to a mixture comprising a base and an organic solvent, said organic solvent being as defined above and said base being selected from potassium carbonate, sodium carbonate, cesium carbonate, trisodium phosphate, and potassium fluoride, so that a compound of the formula (I) is produced, any excess base being eliminated from the reaction mixture prior to recovery of the product (I).

In one aspect, the process comprises adding a solution of a compound of the formula (II) in a mixture of a halonitromethane and an organic solvent, said organic solvent being selected from acetone, dimethylformamide, dimethylacetamide, acetonitrile, dimethylsulfoxide, N-methylpyrrolidone and dimethoxyethane, 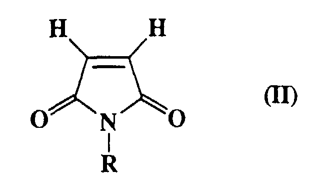 wherein R is defined for formula (I),
to a mixture of a base in an organic solvent, said organic solvent being as defined above and the base being selected from potassium carbonate, sodium carbonate, cesium carbonate, trisodium phosphate, and potassium fluoride, so that a compound of the formula (I) is produced, any excess base being eliminated from the reaction mixture prior to recovery of the product (I).
Preferably R is C₁-C₆ alkyl, C₃-C₆ cycloalkyl or benzyl, the phenyl moiety of the benzyl group being optionally substituted by 1 or 2 substituents as defined above.

Preferably, R is C₁-C₆ alkyl or benzyl.

Most preferably, R is benzyl.

Preferably, the halonitromethane is chloronitromethane or bromonitromethane, most preferably bromonitromethane.

Bromonitromethane is conveniently purchased as a solution in a stabilizing amount of an inert organic solvent, such as toluene, and preferably as a solution in from 25 to 50%w/w toluene. The presence of the inert solvent, such as toluene, does not significantly affect the yield of the compound (I), so that the solution of bromonitromethane in the inert solvent can be used as such in the process of the invention.

Mixtures of the stated organic solvents, and of the bases, may also be used.

Preferably, the ratio of the volume of the organic solvent in the mixture of the base and the organic solvent : the volume of the organic solvent in the solution of the compound (II) and bromonitromethane is from approximately 1:1 to 7:1, preferably from 1:1 to 4:1.

Preferably, the compound (II) and the halonitromethane are used in a molar ratio of from 1:1 to 1:1.25. Most preferably, the molar ratio is about 1:1.

Typically, the solution of the compound (II) in the mixture of the halonitromethane and organic solvent is slowly added to the base/solvent mixture, e.g. over a period of from 30 minutes to 4 hours.

Preferably, the mixture of the base and the organic solvent contains up to 5%, more preferably 1-3%, by volume water based on the total volume of the organic solvent(s).

In one preferred aspect, the organic solvent is dimethylformamide and the base is potassium carbonate.

In another preferred aspect, the organic solvent is dimethylsulfoxide and the base is potassium carbonate.

The process is preferably carried out at a temperature of from 5 to 50°C, preferably from 20°C to 30°C, and most preferably at ambient temperature.

Preferably, any excess base is eliminated from the reaction mixture by filtration, or by neutralisation with an acid. Most preferably, any excess base is removed by neutralisation with acetic or dilute hydrochloric acid.

Preferably, the particle size of the base is less than 75, more preferably less than 45, microns.

Whilst the process as described produces the exo compound (I) in good to excellent yield it is believed that a portion of (I) is produced via the epimerisation of the endo isomer (III), which is also produced in this process:-

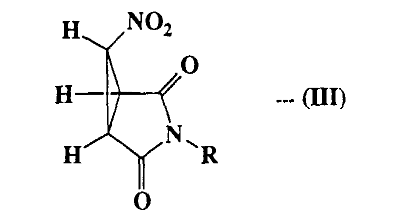

R is as defined for formula (I).

The following Examples illustrate the high yields obtainable by the process of the present invention :gA=grams of activity.

### Example 1

### 1α, 5α, 6α-3-Benzyl-6-nitro-2,4-dioxo-3-azabicyclo[3.1.0]hexane.

To a stirred slurry of potassium carbonate (7.5g;0.054 mole) in acetone (40 ml) and water (1 ml) at ambient temperature was added dropwise, over a period of about 2 hours, a solution of bromonitromethane (5 g - 4.48gA, 0.032 mole) and N-benzylmaleimide (5 g; 0.026 mole) in acetone (40 ml).

When reaction was complete (in situ yield 61%), powdered molecular sieves (10 g) were added and the solvent exchanged with toluene by constant volume distillation. The resulting slurry was filtered to remove unreacted potassium carbonate/sieves/tar and the filter cake washed with toluene.

The combined toluene filtrates were washed with dilute hydrochloric acid (2M), then concentrated under reduced pressure to approximately 20 ml then cooled to 0-5°C. The desired product was subsequently isolated by filtration and dried in vacuo to yield the title compound (3.03gA, 46%) as a white to pale yellow crystalline solid, m.p. 115°C. NMR (CDCl₃): δ 3.34 (s,2H), 4.46 (s,1H), 4.53 (s, 2H), 7.3 (s, 5H).

### Example 2

### 1α, 5α, 6α-3-Benzyl-6-nitro-2,4-dioxo-3-azabicyclo [3.1.0]hexane.

To a stirred slurry of potassium carbonate (7.5 g, 0.054 mole) in dimethylformamide (40 ml) and water (1 ml) at ambient temperature was added dropwise, over a period of about 2 hours, a solution of bromonitromethane (5 g - 4.48gA, 0.032 mole) and N-benzylmaleimide (5 g; 0.026 mole) in dimethylformamide (40 ml).

When the reaction was complete (in situ 72%), the excess potassium carbonate was neutralised by the addition of acetic acid (4.68 g, 0.078 mole) and then water (160 ml) was added. The resulting precipitate was isolated by filtration and dried in vacuo to yield the title compound (4.2 gA, 66%) as an off-white/light brown solid, m.p. 114°C. NMR (CDCl₃):δ 3.34 (s, 2H), 4.46 (s, 1 H), 4.53 (s, 2H), 7.3 (s, 5H).

### Example 3

### 1α, 5α, 6α-3-Benzyl-6-nitro-2.4-dioxo-3-azabicyclo [3.1.0] hexane.

To a stirred slurry of potassium carbonate (7.5 g, 0.054 mole) in dimethylformamide (40 ml) and water (1 ml) at ambient temperature was added dropwise, over a period of about 2 hours, a solution of bromonitromethane (5 g - 4.48gA, 0.032 mole) and N-benzylmaleimide (5 g; 0.026 mole) in dimethylformamide (40 ml).

When the reaction was complete, the excess potassium carbonate was removed by filtration and then water (160 ml) was added.. The resulting precipitate was isolated by filtration and dried in vacuo to yield the title compound (3.5gA, 54%) as a light brown solid, m.p. 116-117°C. NMR (CDCl₃) : δ 3.34 (s, 2H), 4.46 (s, 1 H), 4.53 (s, 2H), 7.3 (s, 5H).

### Examples 4-17

1α, 5α, 6α-3-Benzyl-6-nitro-2,4-dioxo-3-azabicyclo [3,1,0]hexane was prepared similarly to the procedure of Example 1 using N-benzylmaleimide and bromonitromethane in the presence of water and using the stated base/solvent combinations. Although the title compound was not actually isolated in these Examples, the in situ yields of it were measured using hplc following the end of reaction. The isolated yields will be about 5 -15% lower than the in situ yields.

| Example No | Base | Solvent | Molar Ratio NBM:BNM | Water (by vol. based on the total vol.of the organic solvent) | In Situ Yield |
|---|---|---|---|---|---|
| 4 | Pot.Carbonate | Acetonitrile | 1:1.25 | 1.25% | 60% |
| 5 | Pot.Carbonate | DMSO | 1:1.25 | 1.25% | 69% |
| 6 | Pot.Carbonate | DMF | 1:1 | 1.25% | 74% |
| 7 | Pot. Carbonate | DMF | 1:1 | 1.25% | 75% |
| 8 | Pot.Carbonate | DMAC | 1:1:25 | 1.25% | 66% |
| 9 | Pot.Carbonate | NMP | 1:1.25 | 1.25% | 68% |
| 10 | Pot.Carbonate | DME | 1:1.25 | 1.25% | 54% |
| 11 | Cesium Carbonate | Acetone | 1:1.25 | 0.5% | 63% |
| 12 | Cesium Carbonate | DMF | 1:1.25 | 1.25% | 78% |
| 13 | Sodium Carbonate | DMF | 1:1.25 | 1.25% | 61% |
| 14 | Trisodium Phosphate | DMF | 1:1 | 1.25% | 58% |
| 15 | Pot. Fluoride | Acetonitrile | 1:1.25 | 1.25% | 47% |
| 16 | Pot. Fluoride | Acetone | 1:1.25 | 1% | 54% |
| 17 | Pot. Fluoride | DMF | 1:1.25 | 1.25% | 68% |

### Hplc Method:

- Column:: Waters "Novapak" C18 15 cm x 3.9 mm i.d.
- Mobile Phase:: 60:40 0.02 M aqueous sodium dihydrogen phosphate:acetonitrile.
- Flow rate:: 1.0 ml. min ⁻¹l.
UV detection at 220 nm
Approx. retention times:
N-Benzylmaleimide 3.47 minutes.
Bromonitromethane 1.88 minutes.
Title compound 4.59 minutes.
DMSO = Dimethylsulfoxide. DMF = dimethylformamide.
DMAC = dimethylacetamide. NMP = N-methylpyrrolidinone.
DME = dimethoxyethane.
NBM = N-benzylmaleimide. BNM = Bromonitromethane. Pot. = potassium.

### Example 18

To a stirred slurry of potassium carbonate (30g, 0.2136 mole) in DMF (140 ml) and water (4 ml) at ambient temperature was added dropwise, over a period of about 2 hours, a solution of bromonitromethane [36.2g of a 47.5% w/w solution in toluene (about 20 ml), equivalent to 17.19 gA, 0.123 mole] and N-benzylmaleimide (20g, 0.1068 mole) in DMF (20 ml). When the reaction was complete (in situ yield 75%) the excess potassium carbonate was neutralised via the addition of acetic acid (19.4 ml, 20.35 g, 0.34 mole) and then water (160 ml) was added.

The resulting precipitate was isolated by filtration, washed with water (3 x 40 ml) and then isopropanol (3 x 20 ml) to give the title compound (17.54 gA, 66.7%).
NMR (CDCl₃) : δ 3.34 (s,2H), 4.46 (s,1H), 4.53 (s,2H), 7.3 (s,5H).

In the above Examples, the particle size of the bases used was less than 75 microns.

## Claims

1. A process for preparing a compound of the formula (I):- 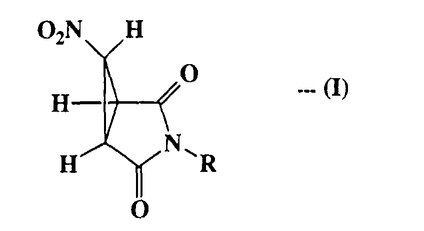 wherein R is C₁-C₆ alkyl, C₃-C₆ cycloalkyl or benzyl, and wherein the phenyl moiety of said benzyl group is optionally substituted by one or more substituents each independently selected from halo, nitro, C₁-C₆ alkyl, C₁-C₆ alkoxy, amino and trifluoromethyl.
which comprises adding a solution comprising a compound of the formula (II), a halonitromethane and an organic solvent selected from acetone, dimethylformamide, dimethylacetamide, acetonitrile, dimethylsulfoxide, N-methylpyrrolidinone and dimethoxyethane, 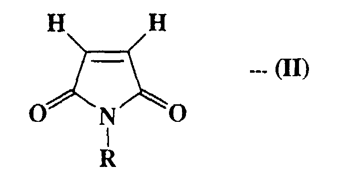 wherein R is as defined above,
to a mixture comprising a base and an organic solvent, said organic solvent being as defined above and said base being selected from potassium carbonate, sodium carbonate, cesium carbonate, trisodium phosphate, and potassium fluoride, so that a compound of the formula (I) is produced, any excess base being eliminated from the reaction mixture prior to recovery of the product (I).

2. A process according to claim 1, which comprises adding a solution of a compound of the formula (II) as defined in claim 1 in a mixture of a halonitromethane and an organic solvent, said organic solvent being selected from acetone, dimethylformamide, dimethylacetamide, acetonitrile, dimethylsulfoxide, N-methylpyrrolidone and dimethoxyethane, to a mixture of a base in an organic solvent, said organic solvent being as defined above and the base being selected from potassium carbonate, sodium carbonate, cesium carbonate, trisodium phosphate, and potassium fluoride, so that a compound of the formula (I) is produced, any excess base being eliminated from the reaction mixture prior to recovery of the product (I).

3. A process according to claim 1 or 2, wherein R is C₁-C₆ alkyl or benzyl..

4. A process according to claim 3, wherein R is benzyl.

5. A process according to any one of the preceding claims, wherein the halonitromethane is chloronitromethane or bromonitromethane.

6. A process according to claim 5, wherein the halonitromethane is bromonitromethane.

7. A process according to claim 6, where the bromonitromethane is used as a solution in a stabilising amount of an inert organic solvent.

8. A process according to claim 7, wherein the bromonitromethane is used as a solution containing from 25 to 50% w/w toluene.

9. A process according to any one of the preceding claims, wherein the compound (II) and the halonitromethane are used in a molar ratio of from 1:1 to 1:1.25.

10. A process according to claim 8, wherein the molar ratio is about 1:1.

11. A process according to any one of the preceding claims, wherein the mixture of the base and the organic solvent contains up to 5% by volume water based on the total volume of the organic solvent(s).

12. A process according to claim 10, wherein the mixture contains from 1-3% by volume water.

13. A process according to any one of the preceding claims, wherein the organic solvent is dimethylformamide and the base is potassium carbonate.

14. A process according to any one of claims 1 to 12, wherein the organic solvent is dimethylsulfoxide and the base is potassium carbonate.

15. A process according to any one of the preceding claims, which is carried out at a temperature of from 5 to 50°C.

16. A process according to claim 14, which is carried out at a temperature of from 20° C to 30° C.

17. A process as claimed in claim 16, which is carried out at ambient temperature.

18. A process according to any one of the preceding claims, wherein any excess base is eliminated from the reaction mixture either by filtration, or by neutralisation with an acid.

19. A process according to claim 18, wherein any excess base is removed by neutralisation with acetic or dilute hydrochloric acid.

20. A process according to any one of the preceding claims, wherein the particle size of the base is less than 75 microns.

21. A process according to any one of the preceding claims wherein the solution of the compound (II) in the mixture of the halonitromethane and organic solvent is slowly added to the base/solvent mixture.

22. A process according to any one of the preceding claims wherein the ratio of the volume of the organic solvent in the mixture of the base and organic solvent : the volume of the organic solvent in the solution of compound (II) and bromonitromethane is from approximately 1:1 to 7:1.

## Patentansprüche

1. Verfahren zur Herstellung einer Verbindung der Formel (I): 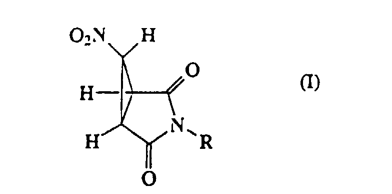 worin R C₁-C₆-Alkyl, C₃-C₆-Cycloalkyl oder Benzyl darstellt und wobei die Phenyleinheit der Benzylgruppe gegebenenfalls mit einem oder mehreren Substituenten, jeweils unabhängig voneinander ausgewählt aus Halogen, Nitro, C₁-C₆-Alkyl, C₁-C₆-Alkoxy, Amino und Trifluormethyl, substituiert ist,
das Zugeben einer Lösung, umfassend eine Verbindung der Formel (II), ein Halogennitromethan und ein organisches Lösungsmittel, ausgewählt aus Aceton, Dimethylformamid, Dimethylacetamid, Acetonitril, Dimethylsulfoxid, N-Methylpyrrolidinon und Dimethoxyethan, 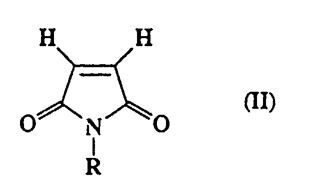 worin R wie vorstehend definiert ist,
zu einem Gemisch, umfassend eine Base und ein organisches Lösungsmittel, wobei das organische Lösungsmittel wie vorstehend definiert ist und die Base aus Kaliumcarbonat, Natriumcarbonat, Cäsiumcarbonat, Trinatriumphosphat und Kaliumfluorid ausgewählt ist, umfasst, so dass eine Verbindung der Formel (I) hergestellt wird, wobei jeglicher Überschuss an Base aus dem Reaktionsgemisch vor der Gewinnung des Produkts (I) entfernt wird.

2. Verfahren nach Anspruch 1, das Zugeben einer Lösung einer wie in Anspruch 1 definierten Verbindung der Formel (II) in einem Gemisch aus einem Halogennitromethan und einem organischen Lösungsmittel, wobei das organische Lösungsmittel ausgewählt ist aus Aceton, Dimethylformamid, Dimethylacetamid, Acetonitril, Dimethylsulfoxid, N-Methylpyrrolidon und Dimethoxyethan, zu einem Gemisch einer Base in einem organischen Lösungsmittel, wobei das organische Lösungsmittel wie vorstehend definiert ist und die Base aus Kaliumcarbonat, Natriumcarbonat, Cäsiumcarbonat, Trinatriumphosphat und Kaliumfluorid ausgewählt ist, umfasst, so dass eine Verbindung der Formel (I) hergestellt wird, wobei jeglicher Überschuss an Base vor der Gewinnung des Produkts (I) aus dem Reaktionsgemisch entfernt wird.

3. Verfahren nach Anspruch 1 oder 2, wobei R C₁-C₆-Alkyl oder Benzyl darstellt.

4. Verfahren nach Anspruch 3, wobei R Benzyl darstellt.

5. Verfahren nach einem der vorangehenden Ansprüche, wobei das Halogennitromethan Chlornitromethan oder Bromnitromethan darstellt.

6. Verfahren nach Anspruch 5, wobei das Halogennitromethan Bromnitromethan darstellt.

7. Verfahren nach Anspruch 6, wobei das Bromnitromethan als eine Lösung in einer stabilisierenden Menge eines inerten organischen Lösungsmittels verwendet wird.

8. Verfahren nach Anspruch 7, wobei das Bromnitromethan als eine Lösung, die 25 bis 50% Gewicht/Gewicht Toluol enthält, verwendet wird.

9. Verfahren nach einem der vorangehenden Ansprüche, wobei die Verbindung (II) und das Halogennitromethan in einem Molverhältnis von 1:1 bis 1:1,25 verwendet werden.

10. Verfahren nach Anspruch 8, wobei das Molverhältnis etwa 1:1 ist.

11. Verfahren nach einem der vorangehenden Ansprüche, wobei das Gemisch aus der Base und dem organischen Lösungsmittel bis zu 5 Volumen% Wasser, bezogen auf das Gesamtvolumen des/der organischen Lösungsmittel/s enthält.

12. Verfahren nach Anspruch 10, wobei das Gemisch 1-3 Volumen% Wasser enthält.

13. Verfahren nach einem der vorangehenden Ansprüche, wobei das organische Lösungsmittel Dimethylformamid darstellt und die Base Kaliumcarbonat darstellt.

14. Verfahren nach einem der Ansprüche 1 bis 12, wobei das organische Lösungsmittel Dimethylsulfoxid darstellt und die Base Kaliumcarbonat darstellt.

15. Verfahren nach einem der vorangehenden Ansprüche, das bei einer Temperatur von 5 bis 50°C ausgeführt wird.

16. Verfahren nach Anspruch 14, das bei einer Temperatur von 20°C bis 30°C ausgeführt wird.

17. Verfahren nach Anspruch 16, das bei Umgebungstemperatur ausgeführt wird.

18. Verfahren nach einem der vorangehenden Ansprüche, wobei jeder Basenüberschuss aus dem Reaktionsgemisch entweder durch Filtration oder durch Neutralisation mit einer Säure entfernt wird.

19. Verfahren nach Anspruch 18, wobei jeglicher Basenüberschuss durch Neutralisation mit Essigsäure oder verdünnter Chlorwasserstoffsäure entfernt wird.

20. Verfahren nach einem der vorangehenden Ansprüche, wobei die Teilchengröße der Base weniger als 75 Mikrometer beträgt.

21. Verfahren nach einem der vorangehenden Ansprüche, wobei die Lösung der Verbindung (II) in dem Gemisch aus dem Halogennitromethan und dem organischen Lösungsmittel langsam zu dem Base/Lösungsmittel-Gemisch gegeben wird.

22. Verfahren nach einem der vorangehenden Ansprüche, wobei das Verhältnis des Volumens des organischen Lösungsmittels in dem Gemisch aus der Base und dem organischen Lösungsmittel : dem Volumen des organischen Lösungsmittels in der Lösung von Verbindung (II) und Bromnitromethan ungefähr 1:1 bis 7:1 ist.

## Revendications

1. Procédé pour la préparation d'un composé de formule (I) : 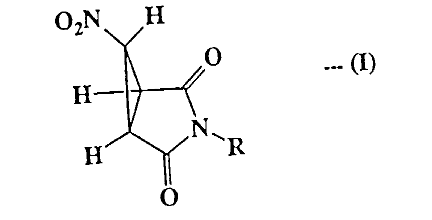 dans laquelle R représente un groupe alkyle en C₁ à C₆, cycloalkyle en C₃ à C₆ ou benzyle, et dans laquelle le groupement phényle dudit groupe benzyle est facultativement substitué avec un ou plusieurs substituants choisis chacun indépendamment entre des substituants halogéno, nitro, alkyle en C₁ à C₆, alkoxy en C₁ à C₆, amino et trifluorométhyle,
qui comprend l'addition d'une solution comprenant un composé de formule (II), un halogénonitrométhane et un solvant organique choisi entre l'acétone, le diméthylformamide, le diméthylacétamide, l'acétonitrile, le diméthylsulfoxyde, la N-méthylpyrrolidinone et le diméthoxy-éthane, 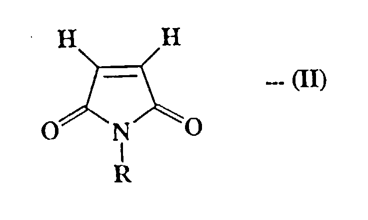 formule dans laquelle R répond à la définition précitée, à un mélange comprenant une base et un solvant organique, ledit solvant organique répondant à la définition précitée et ladite base étant choisie entre le carbonate de potassium, le carbonate de sodium, le carbonate de césium, le phosphate trisodique et le fluorure de potassium, de manière à produire un composé de formule (I), tout excès de base étant éliminé du mélange réactionnel avant de recueillir le produit (I).

2. Procédé suivant la revendication 1, qui comprend l'addition d'une solution d'un composé de formule (II) répondant à la définition suivant la revendication 1 dans un mélange d'un halogénonitrométhane et d'un solvant organique, ledit solvant organique étant choisi entre l'acétone, le diméthylformamide, le diméthylacétamide, l'acétonitrile, le diméthylsulfoxyde, la N-méthylpyrrolidone et le diméthoxy-éthane, à un mélange d'une base dans un solvant organique, ledit solvant organique répondant à la définition précitée et la base étant choisie entre le carbonate de potassium, le carbonate de sodium, le carbonate de césium, le phosphate trisodique et le fluorure de potassium, de manière à produire un composé de formule (I), tout excès de base étant éliminé du mélange réactionnel avant de recueillir le produit (I).

3. Procédé suivant la revendication 1 ou 2, dans lequel R représente un groupe alkyle en C₁ à C₆ ou benzyle.

4. Procédé suivant la revendication 3, dans lequel R représente un groupe benzyle.

5. Procédé suivant l'une quelconque des revendications précédentes, dans lequel l'halogénonitrométhane est le chloronitrométhane ou le bromonitrométhane.

6. Procédé suivant la revendication 5, dans lequel l'halogénonitrométhane est le bromonitrométhane.

7. Procédé suivant la revendication 6, dans lequel le bromonitrométhane est utilisé sous forme d'une solution dans une quantité stabilisante d'un solvant organique inerte.

8. Procédé suivant la revendication 7, dans lequel le bromonitrométhane est utilisé sous forme d'une solution contenant 25 à 50 % en poids/poids de toluène.

9. Procédé suivant l'une quelconque des revendications précédentes, dans lequel le composé (II) et l'halogénonitrométhane sont utilisés en un rapport molaire compris dans l'intervalle de 1:1 à 1:1,25.

10. Procédé suivant la revendication 8, dans lequel le rapport molaire est égal à environ 1:1.

11. Procédé suivant l'une quelconque des revendications précédentes, dans lequel le mélange de la base et du solvant organique contient jusqu'à 5 % en volume d'eau sur la base du volume total du ou des solvants organiques.

12. Procédé suivant la revendication 10, dans lequel le mélange contient 1 à 3 % en volume d'eau.

13. Procédé suivant l'une quelconque des revendications précédentes, dans lequel le solvant organique consiste en diméthylformamide et la base consiste en carbonate de potassium.

14. Procédé suivant l'une quelconque des revendications 1 à 12, dans lequel le solvant organique consiste en diméthylsulfoxyde et la base consiste en carbonate de potassium.

15. Procédé suivant l'une quelconque des revendications précédentes, qui est mis en oeuvre à une température comprise dans l'intervalle de 5 à 50°C.

16. Procédé suivant la revendication 14, qui est mis en oeuvre à une température comprise dans l'intervalle de 20°C à 30°C.

17. Procédé suivant la revendication 16, qui est mis en oeuvre à température ambiante.

18. Procédé suivant l'une quelconque des revendications précédentes, dans lequel tout excès de base est éliminé du mélange réactionnel par filtration ou par neutralisation avec un acide.

19. Procédé suivant la revendication 18, dans lequel tout excès de base est éliminé par neutralisation avec de l'acide acétique ou de l'acide chlorhydrique dilué.

20. Procédé suivant l'une quelconque des revendications précédentes, dans lequel le diamètre de particule de la base est inférieur à 75 micromètres.

21. Procédé suivant l'une quelconque des revendications précédentes, dans lequel la solution du composé (II) dans le mélange de l'halogénonitrométhane et du solvant organique est ajoutée lentement au mélange base/solvant.

22. Procédé suivant l'une quelconque des revendications précédentes, dans lequel le rapport du volume du solvant organique dans le mélange de la base et du solvant organique au volume du solvant organique dans la solution du composé (II) et de bromonitrométhane est compris dans l'intervalle d'approximativement 1:1 à 7:1.
